# EUROPEAN PATENT APPLICATION

(11) **EP 4 600 644 A1**
(43) Date of publication of application: **13.08.2025**
(21) Application number: 23874829.7
(22) Date of filing: 02.10.2023
(51) Int. Cl.: G01N 33/497, A61G 7/05, G01N 27/12

(54) **EXCRETION DETECTION SENSOR DEVICE, EXCRETION DETECTION SENSOR PAD, AND SHEET MEMBER**

(30) Priority: 05.10.2022 JP 2022160632
(71) Applicant: Aba Inc., Yachiyo-chi, Chiba 276-0046 (JP)
(72) Inventor: UI Yoshimi, Funabashi-shi, Chiba 274-0824 (JP); TANIMOTO Kazushiro, Funabashi-shi, Chiba 274-0824 (JP); KOBAYASHI Toshiki, Funabashi-shi, Chiba 274-0824 (JP); KANNO Shouhei, Funabashi-shi, Chiba 274-0824 (JP)
(74) Representative: Plougmann Vingtoft a/s
(86) International application number: PCT/JP2023/035950
(87) International publication number: WO 2024/075702

(57) **Abstract**

An excretion detection sensor device 20 comprises a flexible sensor support 21 and a plurality of odor sensor units 23 disposed on the sensor support 21, wherein at least one of the plurality of odor sensor units 23 has an odor sensor S1 provided inside a recessed portion 24 formed in the sensor support 21.

## Description

### TECHNICAL FIELD

The present invention relates to an excretion detection sensor device, an excretion detection sensor pad, and a seat member.

### BACKGROUND OF THE INVENTION

Conventionally, a system has been known in which air near the buttocks of a care recipient, such as one lying on a bed, is suctioned, and excretion is detected based on a determination result of an odor sensor (see, for example, Patent Document 1). The system includes a tube, a pump, and an odor sensor, where the pump sends air through the tube to the odor sensor. The tube positioned below a seat member disposed under the buttocks of a care recipient.

### PRIOR ART

### PATENT DOCUMENT

[Patent Document 1] Japanese Unexamined Patent Application Publication No. 2019-178890

### SUMMARY OF INVENTION

### PROBLEMS TO BE SOLVED BY THE INVENTION

In conventional technology, since the air near the buttocks is suctioned through the tube, when excrement leaks out, it may be drawn into the system. This leads to issues such as reduced detection accuracy and increased difficulty in maintenance.

Therefore, the present invention has been made in view of these issues, and its object is to provide an excretion detection sensor device, an excretion detection sensor pad, and a seat member with improved detection accuracy and maintainability.

### MEANS FOR SOLVING THE PROBLEM

An excretion detection sensor device according to one aspect of the present invention including: a sensor support having flexibility; and a plurality of odor sensor units disposed on the sensor support, wherein at least one of the plurality of odor sensor units has an odor sensor provided inside a recess formed in the sensor support.

The sensor support may have an elongated belt shape, and the plurality of odor sensor units may be arranged along a longitudinal direction of the sensor support.

At least one of the plurality of odor sensor units may further include: a flexible substrate that supports the odor sensor; and a reinforcing member that is fixed to a region of the flexible substrate that includes at least a portion where the odor sensor is disposed.

The flexible substrate may be provided inside of a member of the sensor support, and the reinforcing member may be a member having higher rigidity than the flexible substrate, and may be fixed to a side of the flexible substrate opposite to a side on which the odor sensor is disposed.

The odor sensor does not need to protrude beyond a surface of the sensor support in a thickness direction of the sensor support when viewed in cross-section.

The odor sensor unit may further include a seal member formed of an air-permeable member and configured to cover an opening of the recess.

The seal member may be made of a moisture-permeable and waterproof material that allows water vapor to pass through but prevents water from passing through.

The excretion detection sensor device according to one aspect of the present invention may further including: a protective layer that is formed thicker than the seal member, has an opening which is larger than the opening of the recess, and is provided on an upper surface of the sensor support so that the seal member is disposed within the opening.

The excretion detection sensor device according to one aspect of the present invention may further including a pressure sensor that is positioned between the plurality of odor sensor units adjacent to each other, and is disposed inside of a member of the sensor support.

An excretion detection sensor pad according to one aspect of the present invention including the excretion detection sensor device described above; and a sheet member larger than the excretion detection sensor device, to which the excretion detection sensor device is attached.

The sheet member may be formed of an air-permeable member and cover an area above the odor sensor units.

The sheet member may be formed of a waterproof member, and include through holes that are positioned above the plurality of odor sensor units.

The sheet member may include a plurality of fixing members for securing the excretion detection sensor device to the sheet member along a longitudinal direction of the excretion detection sensor device.

The sheet member may include through holes formed to be positioned above the odor sensor units, and the fixing members may be provided below the through holes and each form a loop through which the sensor support passes.

The excretion detection sensor device may be a control unit secured to a part of the sensor support, and an interface unit, to which a communication cable or a power supply cable is connected, may be provided in a part of the control unit, the seat member may have a pocket into which the control unit is inserted, and the pocket may have an opening that allows the interface unit to be exposed when the control unit is inserted into the pocket in a first direction, which is an orientation of the control unit in which the excretion detection sensor device is correctly attached to the sheet member; and may prevent the interface unit from being exposed when the control unit is inserted into the pocket in a second direction, which is different from the first direction.

The excretion detection sensor pad may further including: a cover that includes a housing part that accommodates a sheet member to which the excretion detection sensor device is attached, wherein an upper surface side of the housing part may be made of an air-permeable material, and a lower surface side of the housing part may be made of a material with a higher friction coefficient than the material of the upper surface side.

A sheet member according to one aspect of the present invention is the sheet member to which a sensor support, on which a plurality of odor sensor units are formed, is attached, the sheet member including: a fixing member that secures the sensor support, wherein the fixing member may secure the sensor support at a position where the sensor support does not protrude beyond a peripheral edge portion of the sheet member when the sheet member is viewed in a thickness direction of the sheet member.

### EFFECT OF THE INVENTION

According to the present invention, it is possible to provide an excretion detection sensor device and an excretion detection sensor pad with improved detection accuracy and maintainability.

### BRIEF DESCRIPTION OF THE DRAWINGS

FIG. 1 is a diagram showing an example of an information processing system in which an excretion detection sensor device is used.
FIG. 2 is a diagram showing an excretion detection sensor pad disposed on a bed.
FIG. 3 is a diagram illustrating a configuration of the excretion detection sensor pad.
FIG. 4 is a planar view of the excretion detection sensor device.
FIG. 5 is a diagram for explaining a circuit configuration of the excretion detection sensor device.
FIG. 6 is an exploded perspective view illustrating a configuration of a sensor support.
FIG. 7 is a cross-sectional view illustrating a configuration of an odor sensor unit.
FIG. 8 is a schematic view of the odor sensor unit as viewed in the thickness direction.
FIG. 9 is an enlarged sectional view schematically showing a surrounding structure of the odor sensor unit.
FIG. 10 is a cross-sectional view showing a modification of the sensor unit.
FIG. 11 is a perspective view showing another modification of the sensor unit.
FIG. 12 is a view showing a modification of a sheet member.
FIG. 13 is a cross-sectional view illustrating a configuration example in which an odor sensor is disposed in a recess of the sensor support.
FIG. 14A is a cross-sectional view illustrating a configuration example in which a protective layer is provided.
FIG. 14B is a cross-sectional view illustrating another configuration example in which a protective layer is provided.
FIG. 15 is a perspective view showing a modification of the excretion detection sensor pad.
FIG. 16 is a schematic view of the sheet member and the sensor support of FIG. 15.
FIG. 17 is a view showing an example of the arrangement and shape of a fixing member.
FIG. 18 is a diagram illustrating a configuration example of the sheet member according to one embodiment of the present invention.
FIG. 19 is a perspective view showing an example where the information processing system is disposed on a bed while housed in a cover.
FIG. 20 is a schematic cross-sectional view showing a cross-sectional structure of the cover.

### DETAILED DESCRIPTION OF THE INVENTION

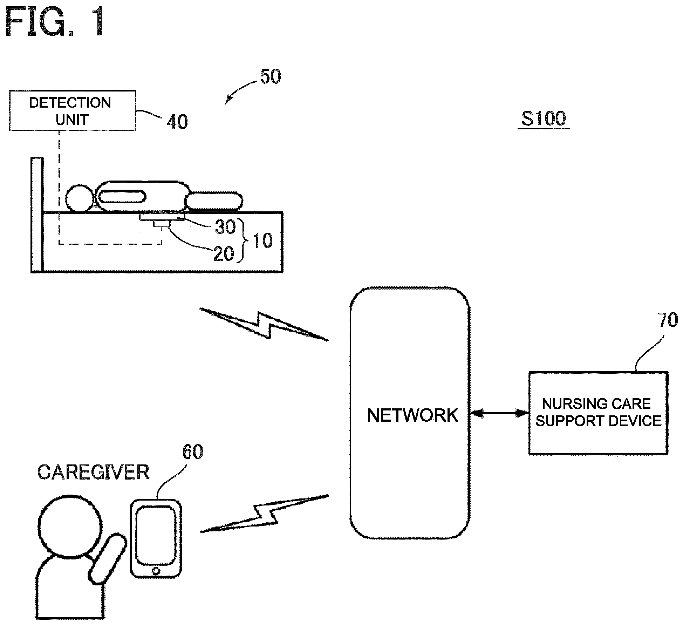

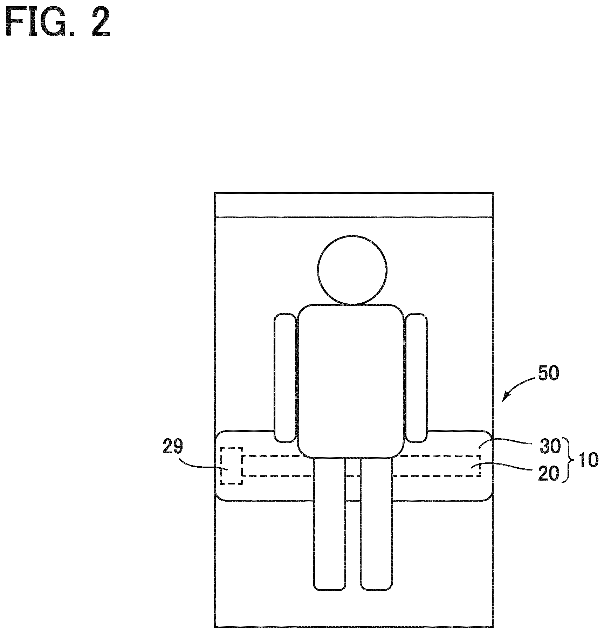

Hereinafter, embodiments of the present invention will be described with reference to the drawings. FIG. 1 is a diagram showing an example of an information processing system S100 in which an excretion detection sensor device is used. FIG. 2 is a view showing an excretion detection sensor pad 10 disposed on a bed.

The information processing system S100 of the present embodiment is a system for supporting the tasks of a caregiver who assists a care recipient. A "care recipient" (hereinafter also referred to as a "recipient") refers to a person, such as an elderly individual in a state of needing nursing care, who cannot manage excrement by himself/herself. The "caregiver" refers to a person who handles tasks such as managing the care recipient's excrement.

The information processing system S100 includes an excretion detection device 50, an information terminal 60, and a nursing care support device 70. The excretion detection device 50, the information terminal 60, and the nursing care support device 70 are connected to a network. The excretion detection device 50 and the information terminal 60 transmit and receive various types of information to and from the nursing care support device 70 via the network.

The excretion detection device 50 detects that the recipient has excreted based on the type of the detected odor, the strength of the odor, or both. The excretion detection device 50 includes the excretion detection sensor pad 10 and a detection unit 40. In the present embodiment, the detection unit 40 is disposed near the excretion detection sensor pad 10, but the detection unit 40 may be hosted on a cloud server or the like via an Internet connection, or may be integrated within the excretion detection sensor pad 10.

As shown in FIG. 2, the excretion detection sensor pad 10 is disposed, for example, on a bed. The excretion detection sensor pad 10 generates an odor signal indicating the intensity or type of odor components. The excretion detection sensor pad 10 outputs the generated signal to the detection unit 40.

The detection unit 40 detects whether the recipient has excreted based on the odor signal generated by the excretion detection sensor pad 10. The detection unit 40 transmits an excretion detection notification indicating that excretion has been detected to the nursing care support device 70.

The information terminal 60 is an information terminal used by a caregiver, and is, for example, a tablet, a smartphone, or a personal computer. The information terminal 60 accepts the input of the content of nursing care tasks performed by the caregiver or displays the information received from the nursing care support device 70.

The nursing care support device 70 is, for example, a computer, creates, for example, various kinds of information useful for supporting nursing care work based on the excretion detection notification transmitted from the detection unit 40, and outputs the created information. The nursing care support device 70 notifies the information terminal 60 that excretion has been detected, for example. As an example, the nursing care support device 70 provides the information terminal 60 a notification about the tasks that the caregiver needs to perform.

With the configuration of the information processing system S100 described above, the caregiver can confirm via the information terminal 60 whether the recipient has excreted, for example. This reduces the burden on the caregiver of having to regularly check the recipient's status to determine if excretion has occurred.

### [Detailed configuration of the excretion detection sensor pad 10]

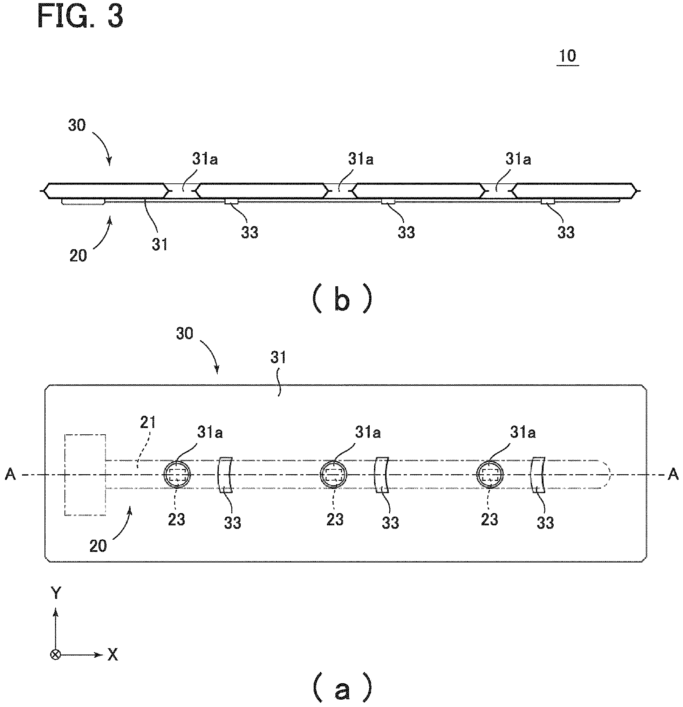

FIG. 3 is a diagram illustrating a configuration of the excretion detection sensor pad 10. FIG. 3(a) is a view of the excretion detection sensor pad 10 as seen from the lower surface side, and FIG. 3(b) is a view schematically showing a cross section taken along a line A-A. The excretion detection sensor pad 10 includes an excretion detection sensor device 20 and a sheet member 30. In FIG. 3(a), the excretion detection sensor device 20 is indicated by the broken line to facilitate understanding of the configuration of the sheet member 30.

The excretion detection sensor pad 10 is a structure in which the excretion detection sensor device 20 is attached to a part of the sheet member 30, and has flexibility as a whole. As shown in FIG. 2, the excretion detection sensor pad 10 is disposed such that the longitudinal direction of the excretion detection sensor pad 10 is the width direction of the bed. As an example, the excretion detection sensor pad 10 is disposed near the recipient's buttocks or thighs of the lower limbs.

The sheet member 30 is a cushion material having flexibility and is disposed such that it covers the excretion detection sensor device 20. The sheet member 30 is not essential to the present invention. However, by providing the sheet member 30 in this manner, the recipient is less likely to feel a sense of discomfort due to the arrangement of the excretion detection sensor device 20, as compared with the case where the excretion detection sensor device 20 is disposed on the bed as a single body.

The excretion detection sensor pad 10 of the present embodiment is not configured to detect air collected through a tube, and as will be described later, an odor sensor unit provided in the excretion detection sensor device 20 detects odor on a bed. According to such a method, the structure to be disposed near the recipient for excretion detection can be made more compact. Hereinafter, each unit will be described in detail.

### (Excretion detection sensor device 20)

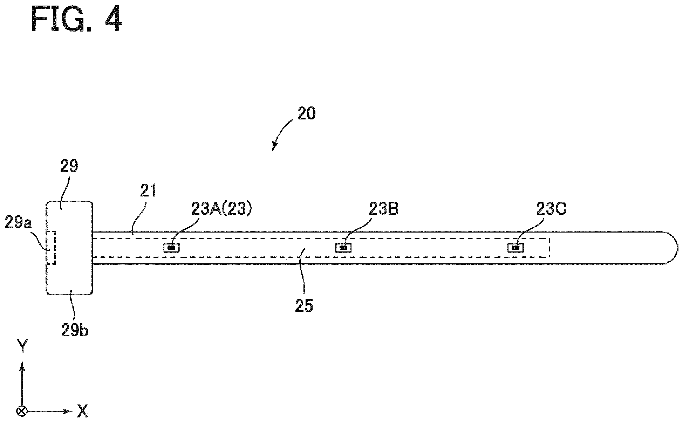

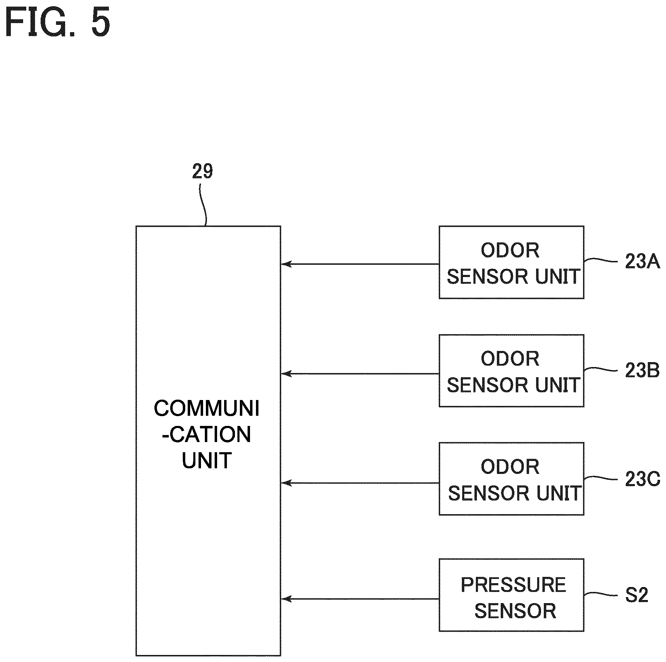

FIG. 4 is a planar view of the excretion detection sensor device 20. FIG. 5 is a diagram showing a circuit configuration of the excretion detection sensor device 20. As shown in FIG. 4, the excretion detection sensor device 20 includes a sensor support 21 and a control unit 29.

The sensor support 21 is a flexible member. The sensor support 21 may have an elongated belt shape or a sheet shape. **In** the present embodiment, it has a belt shape. The length of the sensor support 21 may be any length, but in the present embodiment, for example, the sensor support 21 is formed to be slightly shorter than the width of the bed. The sensor support 21 has, for example, a flat cross-sectional shape. Specifically, the sensor support 21 is preferably flatter than the cross-sectional shape in which the ratio of the width to the thickness is 3: 1.

**In** the case where the sensor support 21 has a belt shape, for example, by disposing the sensor support 21 so as to extend in the width direction of the bed, it is possible to detect odor in a wide range in accordance with turning over or the like of the recipient. **In** addition, the belt-shaped sensor support 21 is less bulky than a sheet and can be easily stored by being wound. Furthermore, the sensor support 21 shown in FIG. 4 does not have a large area like the sheet member 30, for example, and because its area is small, the sensor support 21 has the advantage of being cost-effective.

The sensor support 21 includes a plurality of odor sensor units 23 arranged at predetermined intervals along the longitudinal direction thereof. The sensor support 21 includes an odor sensor unit 23A, an odor sensor unit 23B, and an odor sensor unit 23C as the plurality of odor sensor units 23. Since the three odor sensor units 23 have the same structure, the odor sensor unit 23A will be mainly described below.

The odor sensor unit 23A, the odor sensor unit 23B, and the odor sensor unit 23C each generate an odor signal indicating the intensity or type of odor components, and each output the generated odor signal to the control unit 29 as shown in FIG. 5. The control unit 29 transmits the odor signals to the detection unit 40 (FIG. 1).

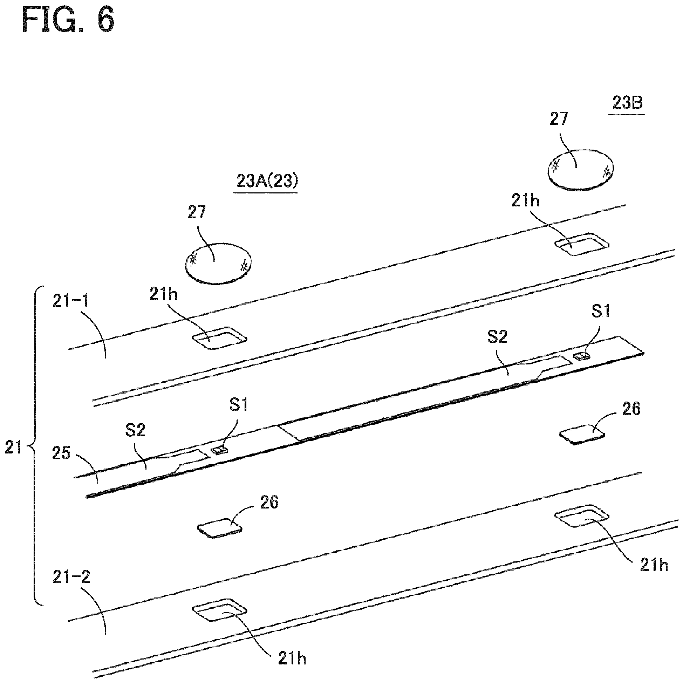

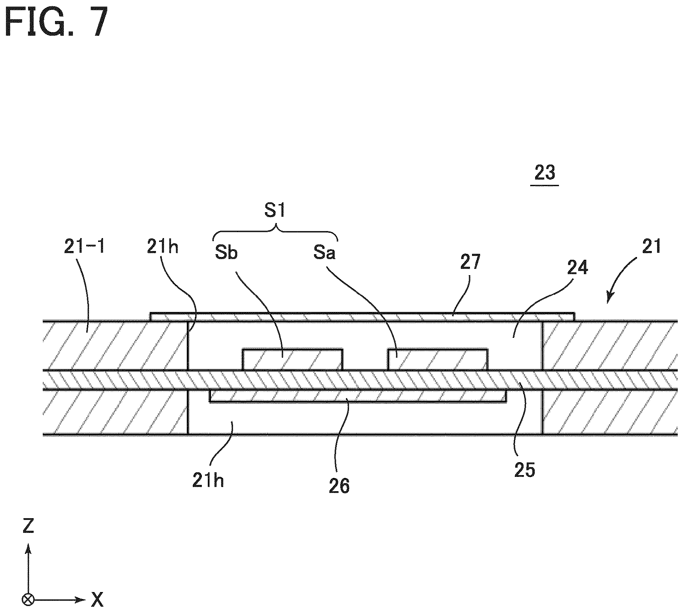

One of the structural features of the excretion detection sensor device 20 of the present embodiment is that the odor sensor is disposed inside a recess 24 formed in the sensor support 21. FIG. 6 is an exploded perspective view illustrating the configuration of the sensor support 21. FIG. 7 is a cross-sectional view illustrating a configuration of the odor sensor unit 23.

### (Sensor support 21)

As shown in FIG. 6, the sensor support 21 includes, as main components, a first band member 21-1, a second band member 21-2, and a flexible substrate 25.

Both the first band member 21-1 and the second band member 21-2 are belt-shaped members having flexibility. The first band member 21-1 and the second band member 21-2 are not particularly limited as long as they are made of a flexible material. In the present embodiment, they are made of resin as an example. Specifically, the first band member 21-1 and the second band member 21-2 are made of rubber, for example.

The first band member 21-1 and the second band member 21-2 may have different shapes, but in the present embodiment, for example, have the same shape. The first band member 21-1 and the second band member 21-2 are stacked on each other, and the flexible substrate 25 is disposed between the members. Hereinafter, the first band member 21-1 will be described as an example, and redundant description of the second band member 21-2 will be omitted.

As shown in FIG. 6, the first band member 21-1 has a plurality of openings 21h arranged at predetermined intervals along the longitudinal direction thereof. The opening 21h is a hole penetrating the first band member 21-1 in the thickness direction. As will be described later, the opening 21h forms the recess 24 (see FIG. 7) in which the odor sensor S1 is disposed. The contour shape of the opening 21h may be any shape, such as a quadrangle, polygon, circle, or ellipse shape. In the present embodiment, the opening 21h is a quadrangle.

In the present embodiment, as shown in FIG. 6, the second band member 21-2 also has a plurality of openings 21h similarly to the first band member 21-1. As an example, the plurality of openings 21h of the second band member 21-2 have the same interval and the same shape as those of the first band member 21-1. It should be noted that the opening 21h of the second band member 21-2 may be omitted.

### (Flexible substrate 25)

The flexible substrate 25 is a substrate having flexibility, and extends along the longitudinal direction of the first band member 21-1 and the second band member 21-2 as shown in FIGS. 4 and 6. The width of the flexible substrate 25 may be equal to or narrower than the widths of the first band member 21-1 and the second band member 21-2, and is narrower than the widths of the first band member 21-1 and the second band member 21-2 in the present embodiment. The length of the flexible substrate 25 may be the same as or shorter than the lengths of the first band member 21-1 and the second band member 21-2, but is shorter than the lengths of the first band member 21-1 and the second band member 21-2 in the present embodiment.

As shown in FIG. 6, the flexible substrate 25 is sandwiched between the first band member 21-1 and the second band member 21-2, and is disposed inside of the member of the sensor support 21 so as not to be exposed to the outside. As shown in FIG. 4, the flexible substrate 25 is connected to the control unit 29.

The control unit 29 includes an interface unit 29a that outputs the odor signals generated by the plurality of odor sensor units 23 to an external device, a housing 29b that houses the interface unit 29a, and a processor. The housing 29b may be shaped flatly to ensure the recipient does not feel discomfort when it is disposed on the bed. The housing 29b may also include a plurality of light-emitting units that illuminate in a predetermined manner to inform a user of, for example, whether a sensor of the odor sensor unit 23 is functioning properly. The interface unit 29a includes, for example, a connector to which cables for communication and power supply are connected. The interface unit 29a may transmit a signal to the external device or receive a signal from the external device by wireless communication.

As shown in FIG. 6, the flexible substrate 25 has a plurality of odor sensors S1 arranged at predetermined intervals along the longitudinal direction thereof. The flexible substrate 25 also has a pressure sensor S2 positioned between the plurality of odor sensor units 23 adjacent to each other. The pressure sensor S2 is provided on the flexible substrate 25, and is disposed inside of the member of the sensor support 21 in the assembled state of the sensor support 21.

### (Odor sensor unit 23)

FIG. 7 is a cross-sectional view illustrating the structure of the odor sensor unit 23. The odor sensor unit 23 includes an odor sensor S1, a reinforcing member 26, and a seal member 27.

The odor sensor S1 is a sensor for detecting the recipient's excretion. The odor sensor S1 is provided inside the recess 24 formed in the sensor support 21. The recess 24 is a recess formed by the opening 21h of the first band member 21-1 and the flexible substrate 25, and has a predetermined depth. Specifically, the recess 24 has a depth corresponding to the thickness of the first band member 21-1.

The odor sensor S1 includes a first sensor Sa and a second sensor Sb. The first sensor Sa and the second sensor Sb are spaced apart from each other by a predetermined interval. The first sensor Sa is, for example, a gas sensor for detecting urination. Specifically, the first sensor Sa reacts to ammonia as one example. The second sensor Sb is, for example, a gas sensor for detecting defecation. Specifically, the second sensor Sb reacts to hydrogen sulfide as one example. It should be noted that the odor sensor S1 may include only one sensor. The odor sensor S1 may include (i) three or more types of sensors or (ii) three or more sensors (which may include a plurality of sensors of the same type).

In the example of FIG. 7, the odor sensor S1 is provided not to protrude beyond the surface of the sensor support 21 in the thickness direction of the sensor support 21 when viewed in cross-section. That is, the odor sensor S1 does not protrude upward beyond the upper surface of the first band member 21-1. According to such a configuration, even if any member comes into contact with the sensor support 21, the possibility that said member comes into contact with the odor sensor S1 is reduced. Therefore, the odor sensor S1 is less likely to be damaged. However, in the present invention, it is not essential that the odor sensor S1 does not protrude beyond the upper surface of the sensor support 21. This will be described with reference to other drawings.

The reinforcing member 26 is a member that reinforces a portion where the odor sensor S1 is disposed. In the case where the odor sensor S1 is mounted on the flexible substrate 25 having flexibility as shown in FIG. 7, there is a possibility that a connection portion between the odor sensor S1 and the wiring pattern of the flexible substrate 25 may be damaged due to bending stress being applied to the connection portion. In view of this, the reinforcing member 26 is provided on the flexible substrate 25 in the present embodiment.

The reinforcing member 26 is, for example, a plate-shaped member. The material of the reinforcing member 26 is not particularly limited, but is, for example, a resin, and specifically, is, for example, a thermosetting material. More specifically, the reinforcing member 26 may be a flat plate made of epoxy. The reinforcing member 26 is, for example, a member having higher rigidity than the flexible substrate 25. In the example of FIG. 7, the reinforcing member 26 is fixed to a side of the flexible substrate 25 opposite to the side on which the odor sensor S1 is disposed. The means for fixing the reinforcing member 26 is not limited to any particular method. For example, an adhesive or a double-sided tape may be used.

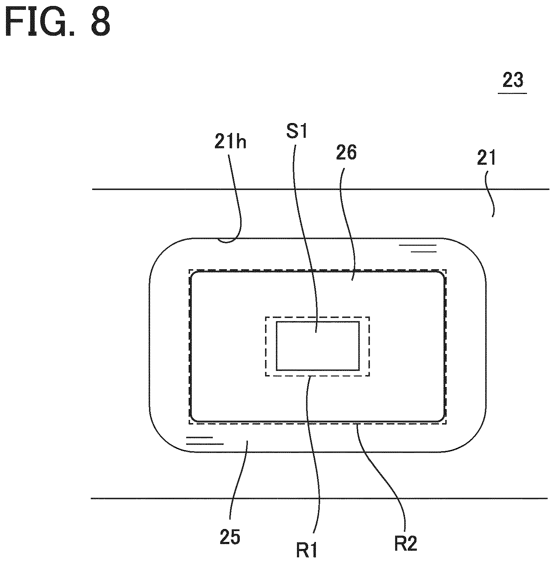

The planar shape of the reinforcing member 26 will be described with reference to FIG. 8. FIG. 8 is a schematic view of the odor sensor unit 23 as viewed in the thickness direction. The odor sensor S1 is schematically illustrated as a quadrilateral component. The contour shape of the reinforcing member 26 may be any shape, such as a quadrangular, polygonal, circular, or elliptical shape. In the present embodiment, it is a quadrangular shape. In the present embodiment, the reinforcing member 26 is fixed to a region R2 that includes at least a portion R1 where the odor sensor S1 is disposed. The reinforcing member 26 may have a shape where a region near the portion R1, where the odor sensor S1 is disposed, is hollowed out.

The size of the reinforcing member 26 is not limited to a specific size. For example, its longitudinal length may be at least twice the longitudinal length of the portion R1 (which corresponds to the width direction of the flexible substrate 25), and its lateral length may be at least twice the lateral length of the portion R1 (which corresponds to the longitudinal direction of the flexible substrate 25). Since the reinforcing member 26 is sufficiently large relative to the portion R1 where the odor sensor S1 is disposed, the reinforcing member 26 can effectively reinforce the odor sensor S1 and its surrounding structure.

In the present embodiment, the contour shape of the reinforcing member 26 is smaller than the opening 21h, as an example. As a specific example, the reinforcing member 26 may be sized such that the distance between the inner periphery of the opening 21h and the outer periphery of the reinforcing member 26 is, for example, 1 mm or more. A configuration where the contour shape of the reinforcing member 26 is larger than that of the opening 21h will be described later with reference to other drawings as a modification.

As shown in FIGS. 6 and 7, the seal member 27 covers an opening of the recess 24 in which the odor sensor S1 is disposed. When the seal member 27 is not provided, excrement or dust may enter the opening 21h. Such intrusion of excrement or dust could damage the odor sensor S1 and its surrounding structure. Therefore, in the present embodiment, the opening of the recess 24 is closed by the seal member 27.

The seal member 27 is in the form of a flexible sheet. The seal member 27 has air permeability so that it does not inhibit the odor sensor S1 from detecting odor. Additionally, it is preferable for the seal member 27 to have waterproof properties to prevent excrement, water during cleaning, or similar substances from entering the opening 21h. As an example, the seal member 27 is preferably made of a moisture-permeable and waterproof material that allows water vapor to pass through but prevents water from passing through.

The waterproof rating is, for example, preferably IPX1 or higher (meaning no harmful effect from vertically falling water droplets), and more preferably IPX5 or higher (meaning no harmful effect from water jets from all directions), according to the waterproof grade in the IP code defined in JIS C0920:2003. Note that IPX1 indicates protection against harmful effects from water droplets falling vertically, while IPX5 indicates protection against harmful effects from water jets sprayed from all directions. The air permeability may be expressed, for example, by a Gurley value (measured in seconds per 100 cc), which represents the time required for 100 cc of air to pass through a 6.45 cm² area under a pressure of 1.27 kPa. In this case, a Gurley value of 1.0 sec/100 cc or less is preferred.

The means for fixing the seal member 27 to the first band member 21-1 is not limited to any particular method. For example, the seal member 27 may be fixed using a double-sided tape, adhesive, heat fusion, or similar means. In FIG. 6, separate seal members 27 are fixed to the respective odor sensor units 23, but for example, the plurality of openings 21h may be covered by the seal member 27 common to the plurality of odor sensor units 23. Specifically, for example, an elongated seal member 27 formed in an elongated shape like the flexible substrate 25 may be disposed so as to cover the plurality of odor sensors S1.

### (Pressure sensor S2)

The pressure sensor S2 provided on the flexible substrate 25 generates a signal corresponding to the pressure applied to the excretion detection sensor device 20 and outputs the generated signal to the control unit 29 (FIG. 5). In the present embodiment, a plurality of pressure sensors S2 are disposed in the longitudinal direction of the sensor support 21 (FIG. 6). As an example, the pressure sensors S2 are disposed within the sensor support 21 and are not exposed to the outside. The shape of the pressure sensor S2 is not specifically limited. In the example shown in FIG. 6, the pressure sensors S2 have a relatively elongated shape extending along the longitudinal direction of the flexible substrate 25. Although not limited to this, the length of each pressure sensor S2 (the length of a pressure receiving portion of the sensor) may be equal to or greater than half the interval between the odor sensor units 23. In another aspect, the pressure sensors S2 may be positioned to overlap the corresponding odor sensors S1.

When such pressure sensors S2 are provided, the detection unit 40 (FIG. 1) may determine that excretion has occurred on the basis of both the output signal of the pressure sensor S2 and the output signal of the odor sensor S1. Specifically, the detection unit 40 may determine that excretion has occurred when the output signal of the pressure sensor S2 exceeds a threshold value corresponding to a predetermined pressure value and the output signal of the odor sensor S1 exceeds a predetermined threshold value. In the determination based on the output signal of the odor sensor S1, it is assumed that a "presence of excretion" may be erroneously determined due to odor components from a sprayed deodorant, even in the absence of a recipient. On the other hand, as described above, according to the method of determining the occurrence of excretion on the basis of both the output signal of the pressure sensor S2 and the output signal of the odor sensor S1, it is possible to reduce the occurrence of erroneous determination.

### (Sheet member 30)

As shown in FIG. 3, the sheet member 30 is a member to which the excretion detection sensor device 20 is attached. Specifically, the excretion detection sensor device 20 is attached to the lower surface of the sheet member 30. In the present embodiment, the sheet member 30 is, for example, larger than the excretion detection sensor device 20 and is formed to have a size that covers the entire excretion detection sensor device 20. The contour shape of the sheet member 30 is, for example, a quadrangle.

The sheet member 30 is placed under the recipient and may be a mat made of an elastic sponge material or an inflatable mat. The sheet member 30 shown in FIG. 3 is, for example, an inflatable mat. The material of the sheet member 30 is not particularly limited, for instance, it may be a waterproof member such as a resin film. In a case where the sheet member 30 is made of a waterproof member, for example, excrement does not penetrate the member, making the sheet member 30 easy to clean.

The sheet member 30 has a plurality of through holes 31a. In the present embodiment, three through holes 31a are formed. Each through hole 31a is positioned to correspond to the odor sensor unit 23 of the excretion detection sensor device 20. When the excretion detection sensor device 20 is attached to a predetermined fixed position on the lower surface of the sheet member 30, each through hole 31a is positioned above the corresponding odor sensor unit 23. By this, the odor sensor unit 23 can detect odors in the air on the upper surface side (that is, the recipient side) of the sheet member 30.

The shape of each through hole 31a is not particularly limited, but is, for example, circular. In one embodiment, the diameter of each through hole 31a, in the planar view as shown in FIG. 3(a), is preferably equal to or less than the width of the sensor support 21 of the excretion detection sensor device 20 (the length of the sensor support 21 in the vertical direction in FIG. 3(a)). With this configuration, the sensor support 21 will be positioned below the through hole 31a. Therefore, even if excrement or the like passes through the through hole 31a and reaches the lower surface 31 side, the excrement is retained on the sensor support 21, preventing the bed from becoming soiled. Even when the through hole 31a has, for example, a polygonal shape, the same effect can be achieved by forming the sensor support 21 to a size that ensures the sensor support 21 is positioned below the through hole 31a.

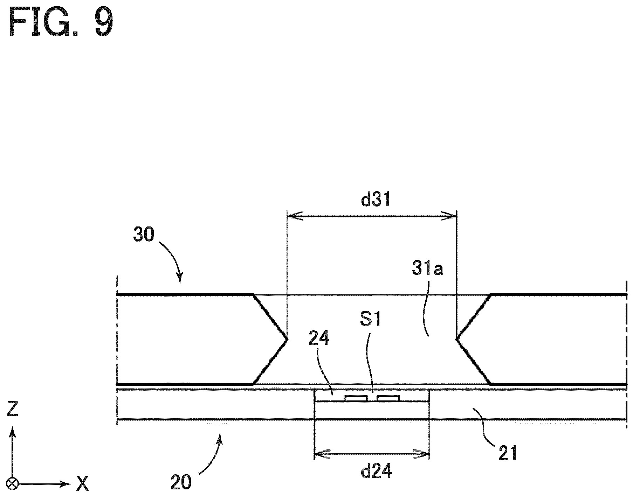

**In** one embodiment, the diameter of the through hole 31a is preferably equal to or greater than the width of the recess 24 in which the odor sensor S1 is disposed, as shown in the planar view of FIG. 3(a). Specifically, as shown in FIG. 9, a diameter d31 of the through hole 31a may be equal to or greater than a width d24 of the recess 24. With this configuration, the sheet member 30 near the odor sensor S1 does not obstruct the odor, allowing for efficient odor detection. Here, the width dimension refers to either the X direction or Y direction measurement (FIG. 3) of the recess 24.

### (Fixing member 33)

As shown in FIG. 3, a plurality of fixing members 33 are formed on the lower surface of the sheet member 30. The plurality of fixing members 33 are formed along the longitudinal direction of the excretion detection sensor device 20. The fixing member 33 is, for example, attached to the lower surface 31 of the sheet member 30 and forms a loop through which the sensor support 21 of the excretion detection sensor device 20 passes. The excretion detection sensor device 20 is secured by threading the sensor support 21 through the plurality of fixing members 33. Although the fixing members 33 with a relatively short length in the X direction are illustrated in FIG. 3, the fixing members 33 may be longer along the X direction.

### (Effects)

In the configuration of the present embodiment described above, the odor sensor S1, provided in the recess 24 formed in the flexible sensor support 21, determines whether excretion has occurred by detecting odors near the buttocks of the recipient on the bed. In particular, because the odor sensor S1 detects odors accumulated in the recess 24, effective odor detection is achieved. Unlike a conventional method that detects air collected through a tube, the present embodiment is configured to detect odors near the recipient by a non-suction method. This prevents issues such as reduced detection accuracy and poor maintainability caused by excrement being drawn into the tube under negative pressure. Therefore, the present embodiment enhances both detection accuracy and the maintainability. Moreover, while a method in which air (odor) drawn through a tube is sent to a predetermined odor sensor and odor is detected by said odor sensor may experience delayed responses depending on the air intake distance. However, the configuration of the present embodiment improves detection responsiveness.

Furthermore, in the configuration of the present embodiment, since it is not the method of detecting the air collected through the tube, the structure disposed on the bed can be made more compact. Additionally, since the odor sensor S1 is disposed within the recess 24 (FIG. 7), it is less likely to be damaged compared to, for example, a configuration where the sensor is disposed on the upper surface of the sensor support 21.

Furthermore, in the configuration of the present embodiment, the reinforcing member 26 is provided in the region that includes at least the portion of the flexible substrate 25 where the odor sensor S1 is disposed. Therefore, it is possible to reduce the occurrence of damage to the odor sensor S1 and its surrounding structure.

### <Modification 1>

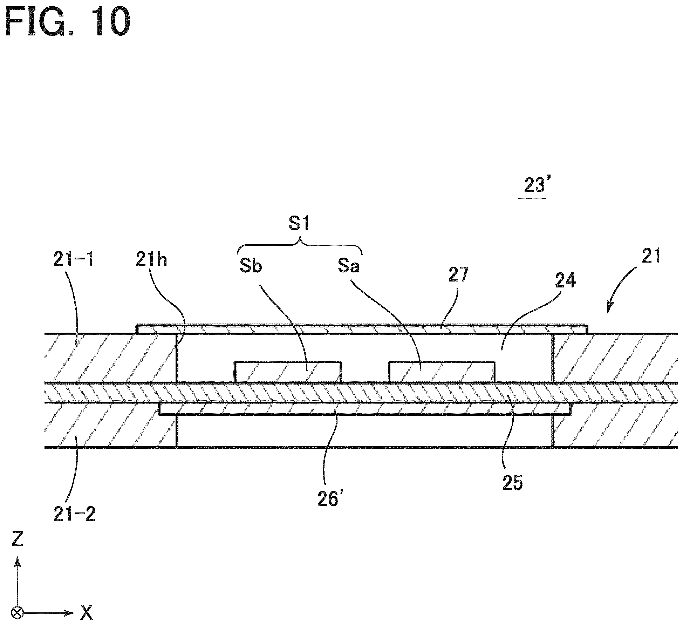

FIG. 10 is a cross-sectional view showing a modification of the sensor unit. A sensor unit 23' in FIG. 10 includes a reinforcing member 26' with a shape different from the reinforcing member 26 of the above-described embodiment. Since other configurations are the same as the above-described embodiment, a detailed description is omitted.

The reinforcing member 26' is formed to have a size larger than that of the opening 21h, with its outer peripheral portion embedded in a part of the sensor support 21. Specifically, as an example, the entire circumference of the reinforcing member 26' is sandwiched between the first band member 21-1 and the second band member 21-2. With this configuration, the flexible substrate 25 is fully supported by the reinforcing member 26' at the opening 21h. As a result, deformation of the flexible substrate 25 within the opening 21h is minimized, making damage to the flexible substrate 25 less likely to occur.

### <Modification 2>

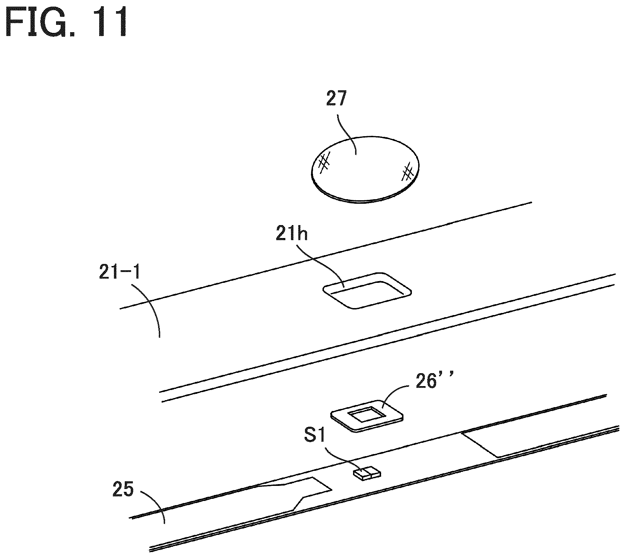

FIG. 11 is a perspective view showing another modification of the sensor unit. As shown in FIG. 11, a reinforcing member 26" may be disposed on the upper surface of the flexible substrate 25. As an example, the reinforcing member 26" has an opening so as not to interfere with the odor sensor S1. With this configuration where the reinforcing member 26" is disposed on the upper surface of the flexible substrate 25, the same effects as those of the embodiment described above can be achieved.

### <Modification 3>

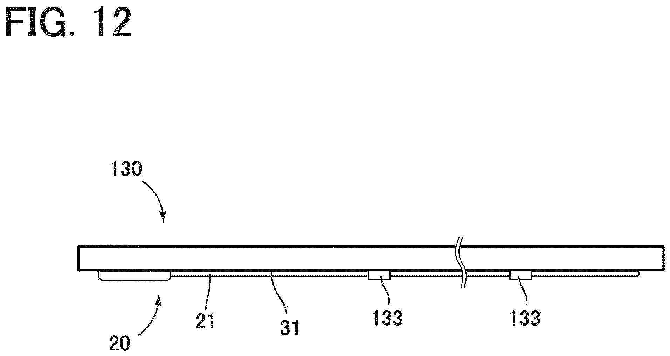

FIG. 12 is a view showing a modification of the sheet member. The sheet member is not necessarily limited to the shape shown in FIG. 3, and may have a configuration as shown in FIG. 12. A sheet member 130 in FIG. 12 is, for example, an elastic, porous material and has air permeability. The sheet member 130 is, for example, a cushion sheet having a predetermined thickness, and may be made of, for example, a synthetic resin sponge material.

The sheet member 130 is not limited to a specific contour shape and may have, for example, a contour shape similar to that of the sheet member 30. As an example, similar to the configuration in FIG. 3, a plurality of fixing members 133 for securing the excretion detection sensor device 20 are provided on the lower surface of the sheet member 130.

Unlike the sheet member 30, the sheet member 130 does not have any through holes. However, since the sheet member 130 itself has air permeability, the excretion detection sensor device 20 can still detect odors from defecation and urination even when the sheet member 130 is disposed such that it covers the excretion detection sensor device 20.

In the above description, the odor sensor S1 is provided in the recess 24 in all the odor sensor units 23. However, it is sufficient for the characteristic structure of one embodiment of the present invention to be provided in at least one of the plurality of odor sensor units 23. The number of odor sensor units 23 is not limited to a plurality, and only one may be provided.

Although the length of the excretion detection sensor pad 10 shown in FIG. 2 is about the same length as the width of the bed, the length of the excretion detection sensor pad 10 can be adjusted as needed. For example, the sheet member 30 may be formed to extend beyond the width of the bed, and during use, the ends of the sheet member 30 may be folded along the sides of the bed. Furthermore, the sheet member 30 can be made long enough so that the distal ends of the folded portions are tucked under the lower surface of a mattress of the bed, for example. The control unit 29 of the excretion detection sensor device 20 can be disposed not only on the top surface of the bed but also on the side surface of the bed.

### <Modification 4>

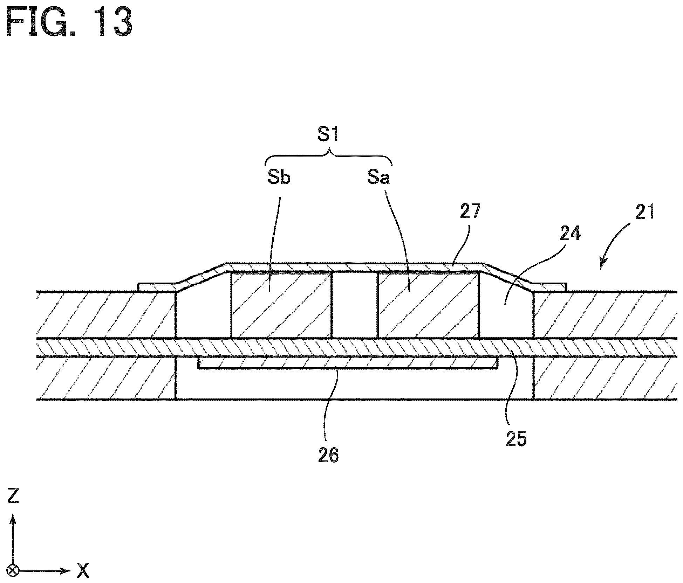

FIG. 13 is a cross-sectional view illustrating a configuration example in which the odor sensor is disposed in the recess of the sensor support. The configuration of FIG. 13 differs from that of FIG. 7 in the shapes of the odor sensor S1 and the seal member 27. Description of the same configuration as that of FIG. 7 will be omitted. As shown in FIG. 13, the odor sensor S1 may protrude beyond the upper surface of the sensor support 21. Even with this configuration, the effects achieved by providing the odor sensor S1 inside the recess 24 of the sensor support 21 can be achieved in the same manner as in the embodiment described above. It should be noted that in the configuration of FIG. 13, the first sensor Sa and the second sensor Sb are included, but as mentioned above, the odor sensor S1 may include only one of these sensors.

### <Modification 5>

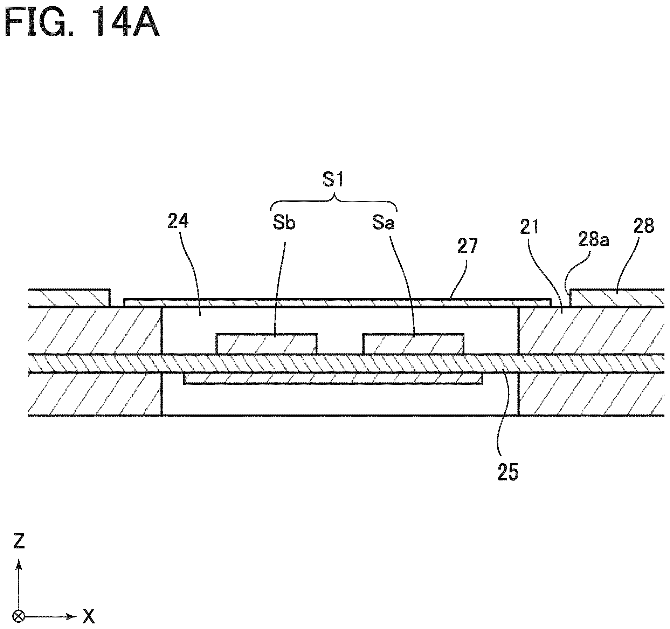

FIG. 14A is a cross-sectional view illustrating a configuration example in which a protective layer is included. Since the configuration of FIG. 14A is the same as the configuration of FIG. 7 except that a protective layer 28 is included, redundant description will be omitted. As shown in FIG. 14A, the protective layer 28 is provided on the upper surface of the sensor support 21.

The protective layer 28 is formed thicker than the seal member 27. The protective layer 28 is a flexible sheet-like member. The protective layer 28 is fixed to the upper surface of the sensor support 21 by adhesive bonding, for example. The protective layer 28 has an opening 28a, which is larger than the opening of the recess 24 of the sensor support 21. In this example, the opening 28a is larger than the outer shape of the seal member 27. The protective layer 28 is disposed such that the seal member 27 is disposed within the opening 28a.

When such a protective layer 28 is provided, the seal member 27 is positioned inside the opening 28a, and the end surface of the seal member 27 does not protrude upward beyond the protective layer 28. This prevents the seal member 27 from being peeled off even if, during operation, the finger of an operator touches the end surface of the seal member 27 or some component comes into contact with the end surface, for example.

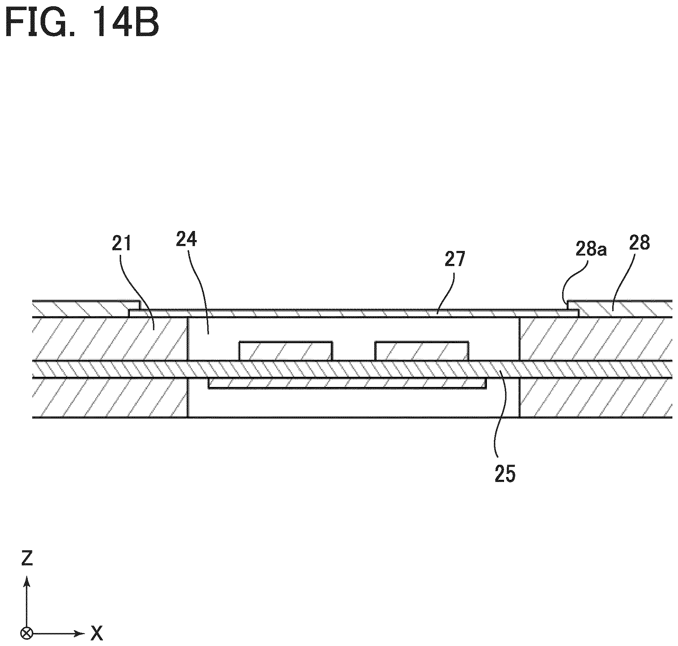

Although the seal member 27 and the protective layer 28 do not overlap each other in the example of FIG. 14A, the protective layer 28 may be provided so as to overlap the peripheral edge portion of the seal member 27 as shown in FIG. 14B. That is, the opening 28a of the protective layer 28 may be formed to be smaller than the outer shape of the seal member 27, and the protective layer 28 may be provided so as to cover the peripheral edge portion of the seal member 27. In such a configuration, the thickness of the protective layer 28 may be thicker than the thickness of the seal member 27, may be the same as the thickness of the seal member 27, or may be thinner than the thickness of the seal member 27. The protective layer 28 may be fixed to the sensor support 21 by an adhesive, or may be fixed to the sensor support 21 by, for example, a double-sided tape attached to one surface of the protective layer 28.

### <Modification 6>

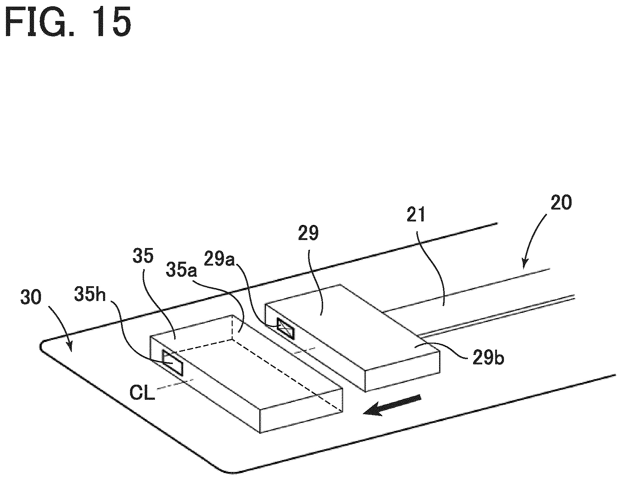

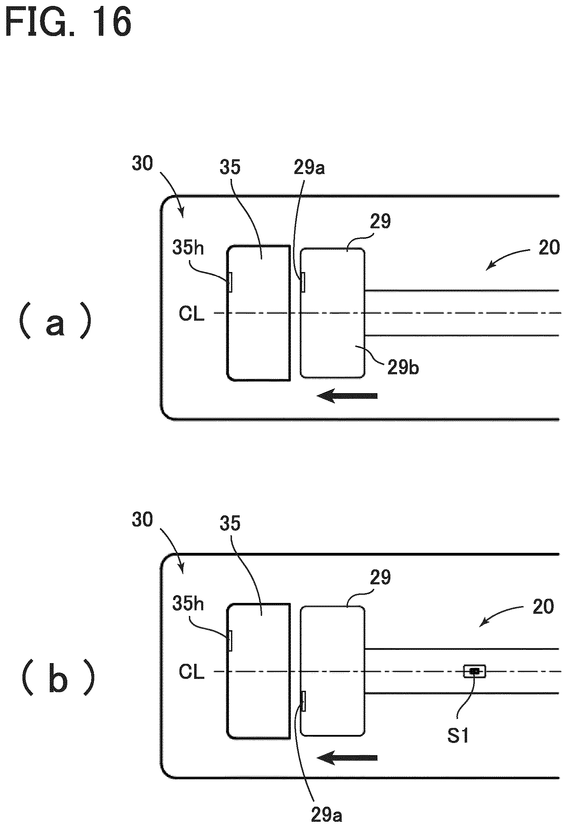

FIG. 15 is a perspective view showing a modification of the excretion detection sensor pad. FIG. 16 is a schematic view of the sheet member and the sensor support of FIG. 15. The excretion detection sensor pad includes the excretion detection sensor device 20 and the sheet member 30 as in the above described embodiment.

The excretion detection sensor device 20 has the control unit 29 secured to a part of the sensor support 21. A part of the control unit 29 is provided with the interface unit 29a to which a communication cable or a power supply cable is connected. As shown in FIGS. 15 and 16, the interface unit 29a is provided at a position offset from a center line CL, which passes through the widthwise center of the housing 29b of the control unit 29, rather than on the center line CL.

The seat member 30 has a pocket 35. The pocket 35 has an inlet 35a, and the control unit 29 is inserted into the pocket 35 from the inlet 35a. An opening 35h is formed in the pocket 35. Similarly to the interface unit 29a, the opening 35h is also positioned offset from the center line CL.

Specifically, the opening 35h is positioned to face the interface unit 29a and expose the interface unit 29a to the outside when the control unit 29 is inserted into the pocket 35 in a first direction (the orientation of the control unit 29 in which the excretion detection sensor device 20 is correctly attached to the seat member 30.) In this way, since the interface unit 29a is exposed to the outside, a cable or the like (not shown) can be connected to the interface unit 29a from the outside of the pocket 35.

As shown in FIG. 16(a), when the excretion detection sensor device 20 is correctly attached to the seat member 30, the interface unit 29a is exposed to the outside of the pocket 35. However, as shown in FIG. 16(b), when the excretion detection sensor device 20 is attached in a reverse orientation and the control unit 29 is inserted into the pocket 35 in a second direction different from the first direction, the interface unit 29a is not exposed to the outside of the pocket 35. This allows the user to recognize that the excretion detection sensor device 20 has been mounted in the reverse orientation. Therefore, with the configurations shown in FIGS. 15 and 16, the problem of failing to properly detect whether excretion has occurred or not caused by an improper orientation of the excretion detection sensor device 20 is less likely to occur.

### <Modification 7>

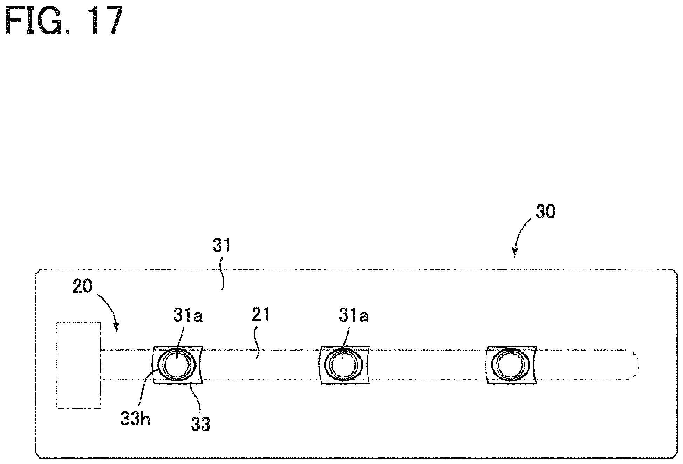

FIG. 17 is a diagram showing an example of the arrangement and shape of the fixing member. As shown in FIG. 17, the fixing member 33 for securing the sensor support 21 of the excretion detection sensor device 20 may be positioned to cover the through hole 31a, rather than being located between the adjacent through holes 31a. This position is below the through hole 31a when the seat member 30, to which the excretion detection sensor device 20 is attached, is disposed on the bed. The fixing member 33 forms a loop through which the sensor support 21 passes. When the fixing member 33 is located in this position, it prevents the sensor support 21 from separating from the lower surface of the sheet member 30 near the through hole 31a. As a result, the odor sensor S1 remains near the through hole 31a, ensuring effective detection of whether excretion has occurred.

The fixing member 33 may or may not have an opening 33h. In the example shown in FIG. 17, the fixing member 33 includes the opening 33h. The opening 33h is positioned such that it overlaps with the through hole 31a when the excretion detection sensor device 20 and the sheet member 30 are viewed in the thickness direction, as shown in FIG. 17. An advantage of having such an opening 33h is that if excrement or the like enters the through hole 31a, cleaning can be easily performed, helping to keep the sheet member 30 clean, for example.

### <Modification 8>

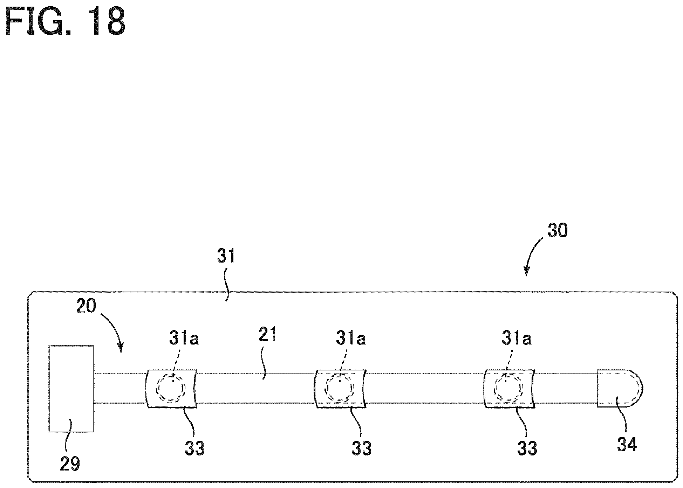

FIG. 18 is a diagram illustrating a configuration example of a sheet member according to one embodiment of the present invention. The sheet member 30 of FIG. 18 has the same configuration as that of FIG. 17, except that (i) the fixing member 33 does not have an opening 33h and (ii) a fixing member 34 is provided at the end portion of the sensor support 21. Therefore, redundant description will be omitted.

A main feature of the sheet member 30 shown in FIG. 18 is that the excretion detection sensor device 20 is configured not to protrude beyond the sheet member 30 when the sheet member 30 is viewed in the thickness direction. Specifically, the fixing members 33 and the fixing member 34 of the sheet member 30 secure the excretion detection sensor device 20 at the position where the excretion detection sensor device 20 does not protrude beyond the peripheral edge portion of the sheet member 30. In this state, as in the above-described embodiment, the odor sensor unit 23 (see FIG. 3) of the sensor support 21 is positioned at the positions of the through holes 31a.

According to the sheet member 30 of one embodiment of the present invention configured in this manner, the excretion detection sensor device 20 can be effectively held in a state where the odor sensor unit 23 of the excretion detection sensor device 20 is exposed to the outside. In addition, since the excretion detection sensor device 20 does not protrude beyond the peripheral portion of the sheet member 30 in a state where the excretion detection sensor device 20 is secured to the sheet member 30, a part of the excretion detection sensor device 20 does not abut against the recipient when the excretion detection sensor device 20 and the sheet member 30 are disposed on the bed. This reduces the recipient's sense of discomfort. In addition, in the configuration of FIG. 18, since the fixing member 34 into which the end portion of the sensor support 21 is inserted is provided, the movement of the sensor support 21 in the longitudinal direction is restricted. This ensures effective positioning of the sensor support 21 in both the width and longitudinal directions.

While FIG. 18 illustrates the configuration where neither the sensor support 21 nor the control unit 29 protrude beyond the sheet member 30, the present invention is not limited thereto. For example, in a configuration in which the control unit 29 is not secured to the sensor support 21, or in a configuration in which the control unit 29 is provided away from the sensor support 21, the sensor support 21 may be provided so as not to protrude beyond the peripheral edge portion of the sheet member 30.

### <Modification 9>

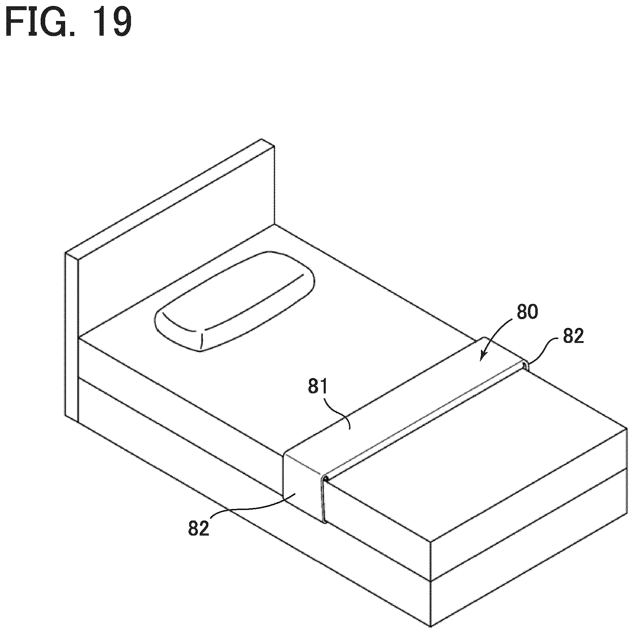

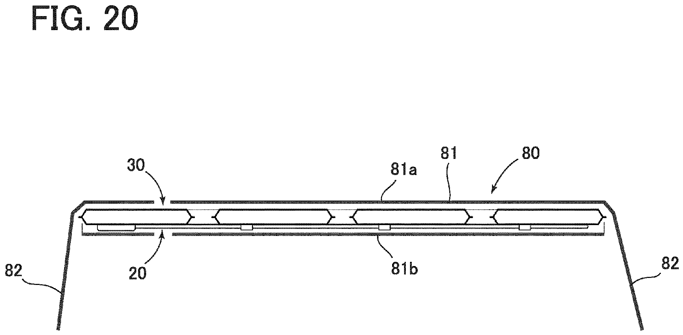

FIG. 19 is a perspective view showing an example where the information processing system is disposed on the bed, enclosed within a cover. FIG. 20 is a schematic cross-sectional view showing a cross-sectional structure of the cover.

The excretion detection sensor pad shown in FIGS. 19 and 20 includes a cover 80. The cover 80 includes a housing part 81 and extending parts 82 at both ends.

The housing part 81 is formed in a cylindrical shape, and accommodates the sheet member 30 to which the excretion detection sensor device 20 is attached. An upper surface member 81a of the housing part 81 is made of air-permeable material. The upper surface member 81a may also be made of a water-repellent material. As an example, a lower surface member 81b of the housing part is made of a material with a higher friction coefficient than the upper surface member 81a. Here, the friction coefficient refers to the static friction coefficient when moving an object disposed on a cloth support, for example. Specifically, the lower surface member 81b is, for example, a sheet material made of polyurethane.

When the lower surface member 81b is made of the material with a high friction coefficient, the position of the excretion detection sensor pad is less likely to shift when the excretion detection sensor pad is disposed on the bed. On the other hand, since the member 81a has air permeability, odors of the excrement reach the inside of the housing part 81 through the member 81a, and the excretion detection sensor device 20 can effectively detect whether excretion has occurred.

The extending parts 82 are provided at both ends of the housing part 81, respectively. Because the extending parts 82 are relatively long, they can be wrapped around the lower surface of the mattress of the bed to stabilize the excretion detection sensor pad, as shown in FIG. 19.

The present disclosure is explained based on the exemplary embodiments. The technical scope of the present disclosure is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

### [Description of Symbols]

10 Excretion detection sensor pad
20 Excretion detection sensor device
21 Sensor support
21-1 First band member
21-2 Second band member
21h Opening
23 Odor sensor unit
24 Recess
25 Flexible substrate
26 Reinforcing member
26' Reinforcing member
26" Reinforcing member
27 Seal member
28 Protective layer
29 Control unit
29a Interface unit
29b Housing
30 Sheet member
31 Lower surface
31a Through hole
33 and 34 Fixing members
35 Pocket
35a Inlet
35h Opening
40 Detection unit
50 Excretion detection device
60 Information terminal
70 Nursing care support device
80 Cover
81 Housing part
82 Extending part
130 Sheet member
CL Center line
S1 Odor sensor
S2 Pressure sensor
S100 Information processing system
Sa First sensor
Sb Second sensor

## Claims

1. An excretion detection sensor device, comprising
a sensor support having flexibility; and
a plurality of odor sensor units disposed on the sensor support, wherein
at least one of the plurality of odor sensor units has an odor sensor provided inside a recess formed in the sensor support.

2. The excretion detection sensor device according to claim 1, wherein
the sensor support has an elongated belt shape, and
the plurality of odor sensor units are arranged along a longitudinal direction of the sensor support.

3. The excretion detection sensor device according to claim 1 or 2, wherein
at least one of the plurality of odor sensor units further includes:
a flexible substrate that supports the odor sensor; and
a reinforcing member that is fixed to a region of the flexible substrate that includes at least a portion where the odor sensor is disposed.

4. The excretion detection sensor device according to claim 3, wherein
the flexible substrate is provided inside of a member of the sensor support, and
the reinforcing member is a member having higher rigidity than the flexible substrate, and is fixed to a side of the flexible substrate opposite to a side on which the odor sensor is disposed.

5. The excretion detection sensor device according to claim 1 or 2, wherein
the odor sensor does not protrude beyond a surface of the sensor support in a thickness direction of the sensor support when viewed in cross-section.

6. The excretion detection sensor device according to claim 1 or 2, wherein
the odor sensor unit further includes
a seal member formed of an air-permeable member and configured to cover an opening of the recess.

7. The excretion detection sensor device according to claim 6, wherein
the seal member is made of a moisture-permeable and waterproof material that allows water vapor to pass through but prevents water from passing through.

8. The excretion detection sensor device according to claim 6, further comprising:
a protective layer that is formed thicker than the seal member, has an opening which is larger than the opening of the recess, and is provided on an upper surface of the sensor support so that the seal member is disposed within the opening.

9. The excretion detection sensor device according to claim 1 or 2, further comprising:
a pressure sensor that is positioned between the plurality of odor sensor units adjacent to each other, and is disposed inside of a member of the sensor support.

10. An excretion detection sensor pad comprising:
the excretion detection sensor device according to claim 1 or 2; and
a sheet member larger than the excretion detection sensor device, to which the excretion detection sensor device is attached.

11. The excretion detection sensor pad according to claim 10, wherein
the sheet member is formed of an air-permeable member and covers an area above the odor sensor units.

12. The excretion detection sensor pad according to claim 10, wherein
the sheet member is formed of a waterproof member, and includes through holes that are positioned above the plurality of odor sensor units.

13. The excretion detection sensor pad according to claim 10, wherein
the sheet member includes
a plurality of fixing members for securing the excretion detection sensor device to the sheet member along a longitudinal direction of the excretion detection sensor device.

14. The excretion detection sensor pad according to claim 13, wherein
the sheet member includes through holes formed to be positioned above the odor sensor units, and
the fixing members are provided below the through holes and each form a loop through which the sensor support passes.

15. The excretion detection sensor pad according to claim 10, wherein
the excretion detection sensor device is a control unit secured to a part of the sensor support, and an interface unit, to which a communication cable or a power supply cable is connected, is provided in a part of the control unit,
the seat member has a pocket into which the control unit is inserted, and
the pocket has an opening that
allows the interface unit to be exposed when the control unit is inserted into the pocket in a first direction, which is an orientation of the control unit in which the excretion detection sensor device is correctly attached to the sheet member; and
prevents the interface unit from being exposed when the control unit is inserted into the pocket in a second direction, which is different from the first direction.

16. The excretion detection sensor pad according to claim 10, further comprising:
a cover that includes a housing part that accommodates a sheet member to which the excretion detection sensor device is attached, wherein
an upper surface side of the housing part is made of an air-permeable material, and
a lower surface side of the housing part is made of a material with a higher friction coefficient than the material of the upper surface side.

17. A sheet member to which a sensor support, on which a plurality of odor sensor units are formed, is attached, the sheet member comprising:
a fixing member that secures the sensor support, wherein
the fixing member secures the sensor support at a position where the sensor support does not protrude beyond a peripheral edge portion of the sheet member when the sheet member is viewed in a thickness direction of the sheet member.
